# EUROPEAN PATENT SPECIFICATION

(11) **EP 3 779 351 B1**
(45) Date of publication and mention of the grant of the patent: **06.09.2023**
(21) Application number: 20186932.8
(22) Date of filing: 21.07.2020
(51) Int. Cl.: G01B 7/04, G01B 7/06, G01N 33/36

(54) **CAPACITIVE SENSOR FOR MEASURING TRANSVERSE GEOMETRIC PARAMETERS OF FLAT RIBBON-SHAPED TEXTILE FIBROUS STRUCTURES AND RELATED METHOD**
KAPAZITIVER SENSOR ZUR MESSUNG TRANSVERSALER GEOMETRISCHER PARAMETER VON FLACHEN BANDFÖRMIGEN TEXTILEN FASERSTRUKTUREN UND ENTSPRECHENDES VERFAHREN
CAPTEUR CAPACITIF POUR LA MESURE DE PARAMÈTRES GÉOMÉTRIQUES TRANSVERSAUX DE STRUCTURES FIBREUSES TEXTILES EN FORME DE RUBAN PLAT ET PROCÉDÉ ASSOCIÉ

(30) Priority: 25.07.2019 CZ 20190486
(43) Date of publication of application: 17.02.2021
(73) Proprietor: VÚTS, a.s., Liberec XI - Ruzodol I 460 01 Liberec (CZ)
(72) Inventor: Svoboda, Miroslav, 460 14 Liberec, Liberec XIV-Ruprechtice (CZ); Braier, Zdenek, 460 10 Liberec, Liberec X-Frantiskov (CZ); Skop, Petr, 460 06 Liberec, Liberec VI-Rochlice (CZ); Zdarek, Pavel, 460 01 Liberec XIII-Nove Pavlovice (CZ)
(74) Representative: Musil, Dobroslav

(56) References cited:
- US-A- 3 371 568
- US-A- 5 122 754
- US-B1- 6 346 819

## Description

### Technical field

The invention relates to a method of measuring transverse geometric parameters of flat ribbon-shaped textile fibrous structures by a capacitive sensor, in which a textile fibrous structure passes through a measuring slot in a plate capacitor.

The invention also relates to a capacitive sensor for measuring transverse geometric parameters of flat ribbon-shaped textile fibrous structures comprising a plate capacitor in which a measuring slot is formed for the passage of a textile fibrous structure.

### Background art

During production, textile yarns are inspected by so-called yarn cleaners, which remove low-quality parts from the yarn. Static yarn samples are inspected in more detail by laboratory instruments, whereby the output is a measuring protocol with the characteristic deficiencies and defects of the yarn.

In order to be able to measure the linear mass of density or diameter of the yarn and to evaluate yarn defects, the linear mass of density or diameter of the yarn is converted into an electrical signal. Two basic measurement principles are used for conversion into an electrical signal - optical and capacitive.

The basic components of an optical sensor are a light source, a light receiver and a diffuser for light scattering. Part of the light from the light source is absorbed by the yarn inside a measuring zone. Consequently, less light falls on the light receiver, the amount of the light captured on the receiver is proportional to the size of the yarn diameter. The disadvantage of the optical sensor is, for example, the impact of ambient lighting conditions on the accuracy of measurement, wear of the components or the unfavorable influence of impurities on the accuracy of measurement. Moreover, it is difficult to measure very small thicknesses of a flat textile fiber by means of the optical sensor.

The basic component of a capacitive sensor is, for example, a twoelectrode air plate capacitor. In the space between two parallel metal electrodes an electric field is created by the action of alternating voltage. If a yarn of variable thickness moves in this field, the capacitance of the plate capacitor also changes. The change in capacitance depends on the amount of fibers in the yarn, on the dielectric constant of the material, but also, for example, on the moisture content in the yarn, which affect the measurement results. For this reason, measurement needs to be made under standard, pre-defined conditions. The disadvantage of the capacitive sensor is the impossibility of determining the transverse geometric parameters of textile fibrous structures, such as the thickness and height of a flat textile fiber, where the height significantly exceeds the thickness.

D1 discloses a device and a method of measuring transverse geometric parameters of yarns with a measuring line with two different capacitive sensors, whereby in each plate capacitor the capacitance is measured and then converted by an RC circuit into a pulse of a width and then sent to an evaluation circuit for calculating geometric parameters.

US 5 122 754 A discloses a system with two concentric capacitors which is used to inspect watermarks in documents. The electrical resistances, together with said capacitances, are used to determine the time constants.

US 6 346 819 B1 discloses a similar device as in the present application wherein the test material is travelling through two identical "precision" capacitors.

The object of the invention is to provide a method of measuring transverse geometric parameters of textile fibrous structures which allows to measure small thicknesses of textile fibers and also textile fibers where the height of the fiber significantly exceeds the thickness of the fiber.

In addition, the object of the invention is to provide a capacitive sensor for measuring transverse geometric parameters of textile fibrous structures, which will remove the disadvantages of the background art.

### Principle of the invention

The object of the present invention is achieved by a method of measuring transverse geometric parameters of flat ribbon-shaped textile fibrous structures, as claimed in claim 1.

In addition, the object of the present invention is achieved by a capacitive sensor for measuring transverse geometric parameters of flat ribbon-shaped textile fibrous structures as claimed in claim 2. With this arrangement, the height of the flat textile fiber and the functional relationship between its thickness and relative permittivity can be determined.

In a preferred variant of embodiment, the distance between the electrodes of the first plate capacitor is different from the distance between the electrodes of the second plate capacitor. The electrode size of the plate capacitor is the same.

### Description of the drawings

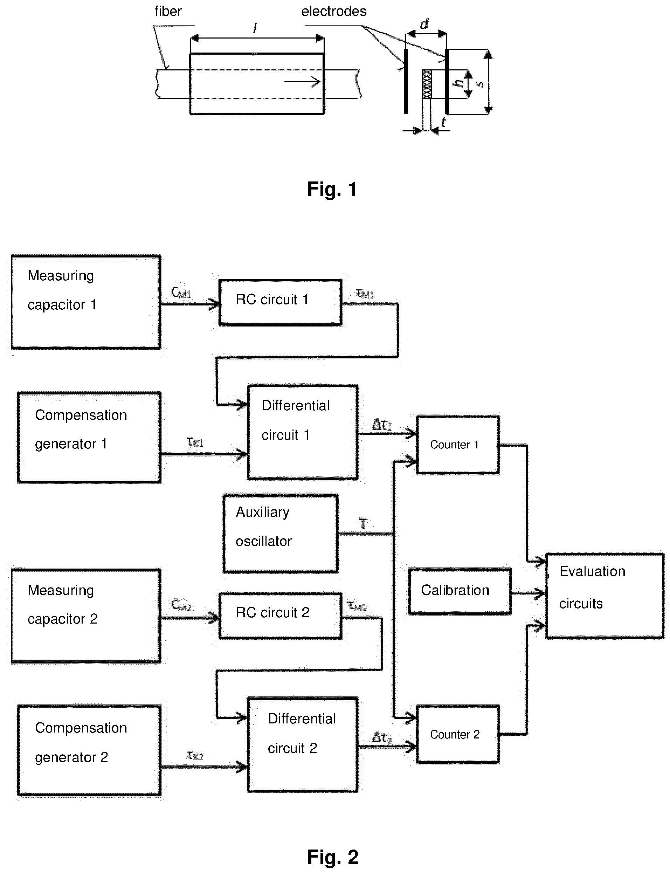

The invention is represented in the enclosed drawings, wherein Fig. 1 shows a plate capacitor in a side and longitudinal view and Fig. 2 shows a schematic block diagram of the capacitive sensor.

### Examples of embodiment

A capacitive sensor comprises a pair of plate capacitors which are arranged one behind the other. Each plate capacitor comprises two conductive electrodes separated by a dielectric with relative permittivity *ε_{r,d}*. In the simplest variant, the dielectric is formed by an air gap in which the measured textile fibrous structure moves.

By placing the textile fiber structure between the electrodes of the plate capacitor, the capacitance C of the plate capacitor changes to the original value of the capacitance *C₀* without the inserted fibrous structure. The magnitude of the change in the capacitance *ΔC* depends on the height of the fibrous structure *h,* on the thickness of the fibrous structure *t***,** on the parameter of relative permittivity *ε_{r,v}* of the fibrous structure and on the distance *d₁, d₂* of the conductive electrodes of the capacitor. The individual parameters of the plate capacitor are shown in Fig. 1. The magnitude of the capacitance of each of the two plate capacitors is sent through computer circuits to an evaluation circuit for determining the geometrical parameters of the textile fibrous material.

The plate capacitors are arranged one behind the other, where the textile fiber structure in the form of a strip passes first through a dielectric in the first plate capacitor and then through a dielectric in the second plate capacitor. In a preferred variant of embodiment, the electrodes of the first and the second plate capacitor have the same size, whereby the distance *d₁* of the electrodes of the first plate capacitor is different than the distance *d₂* of the electrodes of the second plate capacitor. In order for the relative measured change in the capacitance to be as large as possible due to a measurement error, the capacitance of the plate capacitor, and therefore also the dimensions of the electrodes of the plate capacitors, must be as small as possible, but so large that the capacitance can be measured. The value of the capacities is on the order of picofarads with changes on the order of tens of femtofarads. To calibrate the plate capacitors, at least one electrode of each plate capacitor might be mounted slidably reversibly towards the other electrode, either manually or by machine. In an alternative variant, less preferred, the electrodes of the first plate capacitor have a different size than the electrodes of the second plate capacitor.

To measure the value of relative permittivity *ε_{r,v}* of the textile fibrous structure, the capacitive sensor might be provided with a third capacitor, whose electrodes are rotated 90 ° relative to the longitudinal axis of the nonrotated textile fibrous structure.

According to the block diagram in Fig. 1, the output of each plate capacitor is fed to the input of the RC circuit, by which the measured value of the capacitance *C* is converted into a pulse of width τ_{M}, which is directly proportional to the measured capacitance *C.* To measure the change in the capacitance Δ*C*, the capacitive sensor comprises a compensation generator which generates pulses of width τ_{K} corresponding to the width of pulses generated by the plate capacitor without a textile fibrous structure. The outputs of the RC circuit and of the compensation generator are fed to the input of the differential circuit, where from the pulses generated by the plate capacitor and by the compensation generator is obtained the time difference of pulses of width Δτ, which can be converted in a known manner into change in the capacitance Δ*C*. In an alternative variant, the plate capacitor is connected in a resonant circuit with inductance and the resonant frequency is measured.

The output of the differential circuit is fed to the input of the counter which counts the number of pulses *T* of the auxiliary oscillator during the time difference of the pulses of width Δτ and converts this interval into the number of pulses *p.*

For a more accurate calculation of dimensional parameters of a fibrous material, it is necessary to calibrate each plate capacitor and the results of the calibration are included in the calculation of the dimensional parameters of the textile fibrous structure. To calibrate the capacitive sensor, the electrodes of the plate capacitor are moved to a suitable mutual calibration distance d₀ and the compensation generator is set so that the time difference of the pulses of width Δτ is zero, whereby the number of pulses *p* is also zero. Subsequently, the electrodes of the plate capacitor are moved to a measuring distance *d,* the number of pulses *p₀* is measured and the pulse of width τ_{K} generated by the compensation generator is adjusted in such a manner that the indicator of pulses *p* indicates zero again. The calibration distance d₀ and the number of pulses *p₀* are then inserted into the calibration of the evaluation circuits, where calibration constants *k₁* a *k₂* are created.

Measuring the geometric parameters of a textile fibrous structure passing through the first plate capacitor to the second plate capacitor is performed as follows.

In the first plate capacitor, capacitance *C_{M,1}* is measured between the electrodes with the inserted fibrous structure. The first RC circuit, which is connected to the first plate capacitor, converts the measured capacitance *C_{M,1}* of the first plate capacitor with a textile fibrous structure into a pulse of width τ*_{M,1}*. The first compensation generator generates a pulse of width τ*_{K,1}* corresponding to the width of the pulse generated by the first RC circuit of the first plate capacitor without a textile fibrous structure. The difference between the pulse of width *τ_{M,1}* of the first plate capacitor and the pulse of width τ*_{K,1}* of the compensation generator determines the time difference of the pulses of width Δτ*₁*. The first counter counts the number of pulses *p₁* of the coupled auxiliary oscillator generating high speed pulses of width *T* during an interval corresponding to the time difference of pulses of width Δτ₁.

In the second plate capacitor with the inserted fibrous structure, the capacitance *C_{M,2}* is measured, whereby the distance *d₂* of the electrodes of the second plate capacitor is different than the distance *d₁* of the electrodes of the first plate capacitor. The second RC circuit, which is connected to the second plate capacitor, converts the measured capacitance *C_{M,2}* of the second plate capacitor into a pulse of width τ*_{M,2}* and the second compensation generator creates a pulse of width τ*_{K,2}* corresponding to the width of the pulse generated by the second RC circuit of the second plate capacitor without a textile fibrous structure. The difference between the pulse of width τ*_{M,2}* of the second plate capacitor and the pulse of width τ*_{K,2}* of the compensation generator determines the time difference of pulses of width Δτ*₂*. The second counter counts the number of pulses *p₂* of the coupled auxiliary oscillator generating high speed pulses of width *T* during an interval corresponding the time difference of the pulses of width *Δ*τ*₂*.

Information on distance *d₁, d₂* of the electrodes of the plate capacitors, length *l₁*, *l₂* and width *s₁*, *s₂* of the electrodes of the plate capacitors, the number of pulses *p₁, p₂* captured by the counters and the magnitude of the calibration constants *k₁, k₂* for each plate capacitor is sent to an evaluating unit for calculating the height *h* of the textile fibrous structure and the product *α.t,* which is proportional to the thickness of the textile fibrous structure. To calculate the thickness *t* of the textile fibrous structure, it is necessary to know also parameter α, which depends on the relative permittivity ε*_{r,v}* of the textile fibrous structure.

When calculating the thickness *t* of a textile fibrous structure, if the third capacitor is not used, it is necessary to know the value of relative permittivity ε*_{r,v}* of the textile fibrous structure.

## Claims

1. A method of measuring transverse geometric parameters of flat ribbon-shaped textile fibrous structures by a capacitive sensor, in which the textile fibrous structure passes through a measuring slot in a plate capacitor, wherein the textile fibrous structure passes through a measuring slot of a first plate capacitor and subsequently through a measuring slot of a second plate capacitor, whereby the capacitors have different capacitance which is converted by plate capacitor's own RC circuit into a pulse of width τ*_{M}* and compared with an adjustable pulse of width τ*_{K}* generated by plate capacitor's own compensation generator, which corresponds to the pulse width generated by the plate capacitor without a textile fibrous structure, and the time difference of pulses of width Δτ between the pulse of the RC circuit and the pulse of the compensation generator is converted by a counter counting the pulses from an auxiliary oscillator into a number of pulses *p* and information on the number of pulses *p₁, p₂* of each of the two counters is sent to an evaluation circuit for calculating geometric parameters of the textile fibrous structure.

2. A capacitive sensor for measuring transverse geometric parameters of flat ribbon-shaped textile fibrous structures, comprising a first plate capacitor in which a measuring slot is formed for the passage of the textile fibrous structure, wherein a second plate capacitor is arranged behind the first plate capacitor in the direction of movement of the textile fibrous structure, the measuring slot of which is connected to the measuring slot of the first capacitor, whereby the capacitors have different capacitances and the output of each plate capacitor is fed via an RC circuit to an input of a respective differential circuit, to the second input of which the output of a respective compensation generator is connected, whereby the output of the differential circuit is fed to a respective counter to the second input of which an auxiliary oscillator output is connected, whereby the outputs of the counters are fed to the input of a common evaluation circuit.

3. The capacitive sensor according to claim 2, **characterized in that** the first and second capacitors have different electrode distance.

## Patentansprüche

1. Verfahren zum Messen von geometrischen Querparametern von Textilfasergebilden in Form eines flachen Streifens mit Hilfe eines Kapazitätssensors, bei dem das Textilfasergebilde einen Messspalt in einem Plattenkondensator durchgeht, wo das Textilfasergebilde den Messspalt des ersten Plattenkondensators und anschließend den Messspalt des zweiten Plattenkondensators durchgeht, wobei die Kondensatoren unterschiedliche Kapazität aufweisen, die mit Hilfe des RC-Stromkreises zu einem Impuls mit der Breite τ*_{M}* überführt und mit dem einstellbaren Impuls mit der Breite τ*_{K}* verglichen wird, der durch einen Kompensationsgenerator generiert wird, die der Breite des durch den Plattenkondensator ohne Textilfasergebilde generierten Impulses entspricht, und für einen zeitlichen Unterschied von Impulsen mit der Breite Δτ zwischen dem Impuls des RC-Stromkreises und dem Impuls des Kompensationsgenerators durch einen die Impulse aus einem Hilfsoszillator zählenden Zähler zur Impulsanzahl *p* überführt wird und die Informationen über Impulsanzahl *p₁, p₂* von jedem der beiden Zähler in einen Auswertekreis zur Berechnung von geometrischen Parametern des Textilfasergebildes gesendet werden.

2. Kapazitätssensor zum Messen von geometrischen Querparametern von Textilfasergebilden in Form eines flachen Streifens, der den ersten Plattenkondensator aufweist, in dem ein Messspalt zum Durchgang eines Textilfasergebildes gebildet wird, wo hinter dem ersten Plattenkondensator in der Bewegungsrichtung des Textilfasergebildes ein zweiter Plattenkondensator angeordnet ist, dessen Messspalt an den Messspalt des ersten Kondensators anschließt, wobei die Kondensatoren unterschiedliche Kapazität aufweisen und der Ausgang von jedem Plattenkondensator über RC-Stromkreis zum Eingang des entsprechenden Differenzstromkreises zugeführt wird, an dessen anderen Eingang der Ausgang des entsprechenden Kompensationsgenerators angeschlossen ist, wobei der Ausgang des Differenzstromkreises in einen entsprechenden Zähler zugeführt wird, an dessen anderen Eingang der Ausgang des Hilfsoszillators angeschlossen ist, wobei die Ausgänge der Zähler zum Eingang des gemeinsamen Auswertestromkreises zugeführt sind.

3. Kapazitätskondensator nach dem Anspruch 2, **dadurch gekennzeichnet, dass** der erste und der zweite Kondensator einen unterschiedlichen Elektrodenabstand aufweisen.

## Revendications

1. Procédé de mesure de paramètres géométriques transversaux de formations de fibres textiles en forme de bande plate par un capteur capacitif, où la formation de fibres textiles passe par une fente de mesure dans un condensateur à plaques, dans lequel la formation de fibres textiles passe par une fente de mesure d'un premier condensateur à plaques, puis par une fente de mesure d'un second condensateur à plaques, les condensateurs ayant des capacités différentes, converties en une largeur d'impulsion τ*_{M}* par un circuit RC et comparées à une largeur d'impulsion ajustable τ*_{K}* générée par un générateur de compensation, qui correspondent à la largeur d'impulsion générée par le condensateur à plaques sans la formation de fibres textiles, et pour la différence de temps de largeur d'impulsion Δτ entre l'impulsion du circuit RC et l'impulsion du générateur de compensation, le compteur d'impulsions qui compte les impulsions de l'oscillateur auxiliaire est converti en nombre d'impulsions *p* et les informations sur le nombre d'impulsions *p1, p2* de chacun des deux compteurs sont envoyées au circuit d'évaluation qui effectue le calcul des paramètres géométriques de la formation de fibres textiles.

2. Capteur capacitif pour mesure des paramètres géométriques transversaux des formations de fibres textiles ayant la forme d'une bande plate, comprenant un premier condensateur à plaques dans lequel une fente de mesure est formée pour faire passer la formation de fibres textiles, dans lequel un deuxième condensateur à plaques est disposé derrière le premier condensateur à plaques dans la direction du mouvement de la formation de fibres textiles, dont la fente de mesure est reliée à la fente de mesure du premier condensateur à plaques, dans lequel les condensateurs ont des capacités différentes et la sortie de chaque condensateur à plaques est alimentée par un circuit RC à l'entrée du circuit différentiel respectif, à la deuxième entrée duquel est connectée la sortie d'un générateur de compensation respectif, dans lequel la sortie du circuit différentiel est alimentée à un compteur respectif, à la deuxième entrée duquel est connectée la sortie d'un oscillateur auxiliaire, tandis que les sorties des compteurs sont conduites à l'entrée du circuit d'évaluation commun.

3. Le condensateur capacitif selon la revendication 2, **caractérisé en ce que** le premier et le second condensateur ont un espacement différent entre les électrodes.
